# NOUVEAU FASCICULE DE BREVET EUROPEEN

(11) **EP 1 647 262 B2**
(45) Date de publication et mention de la décision concernant l'opposition: **08.02.2012**
(45) Mention de la délivrance du brevet: 05.08.2009
(21) Numéro de dépôt: 05292142.6
(22) Date de dépôt: 13.10.2005
(51) Int. Cl.: A61K 8/40, A61Q 5/06

(54) **Composition comprenant, dans un milieu cosmétiquement acceptable anhydre, des monomères électrophiles et un acide, et son utilisation pour le traitment cosmétique des cheveux**
Wasserfreie kosmetische Zusammensetzung enthaltend elektrophile Monomere und Säure und deren Verwendung zur kosmetischen Behandlung von Haaren
Anhydrous cosmetic composition comprising electrophilic monomers and acid, and use for cosmetic treatment of hair

(30) Priorité: 13.10.2004 FR 0410813
(43) Date de publication de la demande: 19.04.2006
(60) Demande divisionnaire: 09151186.5
(73) Titulaire: L'Oréal, 75008 Paris (FR)
(72) Inventeur: Vic, Gabin, 60280 Venette (FR); Gourlaouen, Luc, 92600 Asnieres (FR); Livoreil, Aude, 75006 Paris (FR); Brun, Gaëlle, 75015 Paris (FR); Giroud, Franck, 92110 Clichy (FR)
(74) Mandataire: Casalonga, Axel

(56) Documents cités:
- WO-A-03/053380
- WO-A1-97//31598
- WO-A1-99//10020
- WO-A2-01//46327
- US-A- 3 840 490
- US-A- 5 964 227
- US-A- 6 001 345

## Description

La présente invention est relative à une composition pour le traitement cosmétique des cheveux comprenant au moins un monomère électrophile et au moins un acide particulier, dans un milieu cosmétiquement acceptable anhydre.

La présente invention concerne également l'utilisation d'une telle composition pour le traitement cosmétique des cheveux, et un procédé de traitement cosmétique mettant en oeuvre une telle composition.

Dans le domaine de la cosmétique, on cherche à modifier les propriétés superficielles des cheveux, notamment pour leur apporter un effet conditionnant comme de la douceur, ou de la brillance. Pour ce faire, on utilise généralement des compositions cosmétiques à base d'agents de conditionnement tels que des silicones ou des polymères ayant une forte affinité pour les cheveux.

Cependant, ces agents de conditionnement ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables.

La Demanderesse a trouvé de manière surprenante qu'en associant un milieu cosmétiquement acceptable anhydre particulier et au moins un acide particulier à au moins un monomère électrophile tel que décrit ci-dessous, il était possible d'obtenir un conditionnement et une brillance améliorés des cheveux, et ce de manière durable.

En effet, une composition comprenant une telle association permet de maintenir la douceur et la brillance apportées à la chevelure par ladite composition, et ceci sans ré-application, même après plusieurs lavages de la chevelure.

L'application d'une composition comprenant une telle association conduit à la formation in situ d'un revêtement lubrifiant et brillant, rémanent notamment aux shampoings.

En outre, les cheveux restent de manière surprenante parfaitement individualisés est peuvent être coiffés sans problème.

L'invention a donc pour objet une composition pour le traitement cosmétique des cheveux comprenant au moins un monomère électrophile 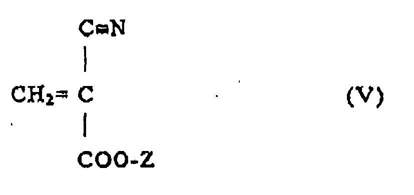 de formule (V) : dans laquelle :

Z=-(CH₂)₇-CH₃,

-CH(CH₃)-(CH₂)₅-CH₃,

-CH₂-CH(C₂H₅)-(CH₂)₃-CH₃,

-(CH₂)₅-CH(CH₃)-CH₃,

(CH₂)₄-CH(C₂H₅)-CH₃,

et au moins un acide organique non-réducteur choisi parmi l'acide acétique, l'acide formique, l'acide propionique, l'acide butyrique, les acides mono-, di- ou trichloroacétiques, et l'acide trifluoroacétique,, dans un milieu cosmétiquement acceptable anhydre choisi parmi les silicones, les huiles minérales, les huiles végétales, les alcanes en C₅-C₁₀, l'acétone, la méthyléthylcétone, le diméthoxyéthane, le diéthoxyéthane, les alcools gras en C₁₀-C₃₀, les amides gras en C₁₀-C₃₀, les esters d'alcool gras en C₁₀-C₃₀ et leurs mélanges.

Un autre objet de la présente invention consiste en l'utilisation de ladite composition pour le traitement cosmétique des cheveux.

L'invention a encore pour objet un procédé de traitement cosmétique des cheveux, mettant en oeuvre ladite composition.

D'autres objets, caractéristiques, aspects et avantages de l'invention apparaîtront encore plus clairement à la lecture de la description et des exemples qui suivent.

Selon l'invention, la composition comprend dans un milieu cosmétiquement acceptable, anhydre, au moins un monomère électrophile de formule (V) et au moins un acide organique non-réducteur tel que défini ci-avant.

Par "non-réducteur", on entend au sens de la présente invention un composé dont le potentiel d'oxydo-réduction standard à 25°C (par rapport à l'électrode à hydrogène) est supérieur à 0 volt.

Par milieu cosmétiquement acceptable, on entend un milieu compatible avec les cheveux.

On entend par « milieu anhydre », un milieu contenant moins de 1 % en poids d'eau par rapport au poids total de la composition.

Le milieu cosmétiquement acceptable est choisi parmi les silicones telles que par exemple les silicones volatiles, les gommes ou huiles de silicones aminés ou non et leurs mélanges ; les huiles minérales ; les huiles végétales telles que par exemple les huiles d'olive, de ricin, de colza, de coprah, de germe de blé, d'amande douce, d'avocat, de macadamia, d'abricot, de carthame, de noix de bancoulier, de camélina, de tamanu, de citron ; ou encore des composés organiques tels que des alcanes en C₅-C₁₀, l'acétone, la méthyléthylcétone, le diméthoxyéthane, le diéthoxyéthane, les alcools gras en C₁₀-C₃₀ tels que par exemple l'alcool laurique, l'alcool cétylique, l'alcool stéarylique et l'alcool béhénylique, les amides gras en C₁₀-C₃₀ tels que part exemple le diéthanolamide laurique, les esters d'alcools gras en C₁₀-C₃₀ tels que par exemple les benzoates d'alcool gras en C₁₀-C₃₀ et leurs mélanges.

De préférence, les composés organiques constituant le milieu sont choisis parmi les composés liquides à la température de 25°C et sous 10⁵ Pa (760mm de Hg).

Le milieu cosmétiquement acceptable utilisé de préférence est choisi parmi les huiles d'olive, de ricin, de colza, de coprah, de germes de blé, d'amande douce, d'avocat, de macadania, d'abricot, de carthame, de noix de bancoulier, de camélina, de tamanu, de citron ; l'huile de polybutène, le mélange cyclopentasiloxane (14,7% en poids)/polydiméthylsiloxane dihydroxylé en positions α et ω (85,3% en poids), ou leurs mélanges.

L'acide particulièrement préféré dans la présente invention est l'acide acétique.

L'acide ou le mélange d'acides tels que décrits ci-dessus, est présent dans les compositions cosmétiques de l'invention, en une concentration comprise entre 0,001 et 20% en poids, de préférence entre 0,002 et 10% en poids, et mieux encore entre 0,005 et 5 % en poids par rapport au poids total de la composition.

Par monomère électrophile, on entend un monomère capable de polymériser par polymérisation anionique en présence d'un agent nucléophile tel que par exemple, les ions hydroxyles (OH⁻) contenus dans l'eau.

Par polymérisation anionique, on entend le mécanisme défini dans l'ouvrage "Advanced Organic Chemistry", Third Edition de Jerry March, pages 151 à 161.

Selon la présente invention, les monomères sont choisis parmi les monomères capables de polymériser sur les fibres kératiniques dans des conditions cosmétiquement acceptables. En particulier, la polymérisation du monomère s'effectue de préférence à une température inférieure ou égale à 80°C, de préférence entre 10 et 80°C, de préférence 20 à 80°C, ce qui n'empêche pas de terminer l'application par un séchage au casque, un brushing ou passage au fer plat ou à friser.

Les compositions mises en oeuvre conformément à l'invention ont généralement une concentration en monomère électrophile selon l'invention comprise entre 0,001 et 80 % en poids, de préférence entre 0,002 et 75 % en poids, plus particulièrement entre 0,1 et 40 % et encore plus préférentiellement entre 1 et 20 % en poids par rapport au poids total de la composition.

On peut également introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges. Les groupements alkyle désignent de préférence des groupements ayant 1 à 6 atomes de carbone.

La quantité d'inhibiteur(s) peut aller de 10 ppm à 20%, et plus préférentiellement de 10 ppm à 5% et encore plus préférentiellement de 10 ppm à 1 % en poids par rapport au poids total de la composition.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols, des protéines, des vitamines, des colorants directs ou d'oxydation, des agents nacrants, des propulseurs, et des épaississants minéraux ou organiques tels que le benzylidène-sorbitol et les N-acylaminoacides.

Ces agents peuvent être éventuellement encapsulés. La capsule peut être de type polycyanoacrylate.

La composition selon l'invention est utilisée sur les cheveux, de préférence en présence d'un agent nucléophile, pour leur traitement cosmétique.

Un procédé de traitement cosmétique selon l'invention comprend l'application d'une composition telle que définie ci-dessus, sur les cheveux, en présence d'un agent nucléophile tel que défini ci-dessous.

Les agents nucléophiles susceptibles d'initier la polymérisation anionique sont des systèmes connus en eux-mêmes, capables de générer un carbanion au contact d'un monomère électrophile. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophile. De façon non limitative, on peut citer comme fonctions nucléophiles les fonctions : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃-, OH⁻, ArNH, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

Les agents nucléophiles particulièrement préférés sont les ions hydroxyle, notamment ceux présents dans l'eau. Cette eau peut être apportée par une humidification préalable des cheveux.

Selon un mode préféré de réalisation, la composition comprenant le monomère électrophile est exempte d'agent nucléophile.

Selon un autre mode préféré de réalisation, l'agent nucléophile est apporté par une seconde composition, appliquée sur ou sous le dépôt formé par l'application de la composition comprenant le monomère électrophile.

Il est également possible, afin de moduler la cinétique de réaction, de préalablement humidifier les cheveux à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des cheveux, par transformation chimique de la matière kératinique.

A titre d'exemple de transformation chimique, on peut citer la réduction des ponts di-sulfure composant en partie la kératine en thiols, avant application de la composition de l'invention. De façon non exhaustive, on peut citer comme réducteurs des ponts di-sulfure composant en partie la kératine, les composés suivants:
- thiosulfate de sodium anhydre,
- métabisulfite de sodium en poudre,
- thiourée,
- sulfite d'ammonium,
- acide thioglycolique,
- acide thiolactique,
- thiolactate d'ammonium,
- mono-thioglycolate de glycérol,
- thioglycolate d'ammonium
- thioglycérol,
- acide 2,5-dihydroxybenzoique,
- di-thioglycolate de diammonium,
- thioglycolate de strontium,
- thioglycolate de calcium,
- formo-sulfoxylate de zinc,
- thioglycolate d'isooctyle,
- dl-cystéine,
- thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter les cheveux avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Ces compositions peuvent se présenter sous la forme de lotion, de spray, de mousse, et être appliquées comme shampoing ou après-shampoing.

Un autre objet de l'invention consiste en un kit comprenant une première composition contenant au moins un monomère électrophile tel que défini ci-dessus et éventuellement au moins un inhibiteur de polymérisation anionique et/ou radicalaire tel que défini ci-dessus, ainsi qu'une deuxième composition comprenant, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀-C₃₀ et leurs mélanges, au moins un acide organique non-réducteur tel que défini ci-dessus.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemple 1 :

On a préparé une crème de brillance selon l'invention (exemple 1) et une crème de brillance comparative (exemple comparatif 1) à partir des ingrédients suivants :

| | Ex. 1 | Ex. Comp. 1 |
|---|---|---|
| 2-cyanoacrylate de N-octyle | 5% | 5% |
| Cyclopentasiloxane | 47,35% | 47,4% |
| Cyclopentasiloxane dimethicone copolyol | 47,4% | 47,4% |
| Acide acétique | 0,05 % | - |
| parfum | 0,2 % | 0,2 % |

### Mode d'application

Des mèches constituées de 2,7g de cheveux moyennement sensibilisés sont humidifiées à l'aide de 1ml d'eau par mèche. 2g de chacune des compositions des exemple 1 et exemple comparatif 1, sont appliqués sur ces mèches humidifiées. Après application, les mèches de cheveux sont séchées au casque 30 minutes à 40°C.

Pour chaque mèche, le toucher et la brillante des cheveux sont évalués par un panel de 10 personnes. Une mèche vierge de même nature est utilisée comme référence.

L'évaluation tactile et visuelle des diverses mèches de cheveux est répétée avec la même procédure après avoir réalisé successivement 5 shampooings commercialisés sous la dénomination DOP camomille.

Les résultats d'évaluation sensorielle sont indiqués dans le tableau suivant :

| Nature du traitement | Ex. 1 | Ex. comp. 1 |
|---|---|---|
| Après application | Douceur 5 | Douceur 3 |
| | Brillance 5 | Brillance 3 |
| Après 5 shampoings | Douceur 4 | Douceur 2 |
| | Brillance 4 | Brillance 2 |

La notation est :
0 = équivalent à la mèche non traitée,
5 = très supérieur à la mèche non traitée.

La douceur et la brillance apportées par la composition selon l'invention sont supérieures à celles apportées par la composition de l'exemple comparatif 1 juste après l'application de la composition. Après 5 shampooings, l'apport de douceur et de brillance est mieux conservé lorsque les mèches sont traitées avec la composition selon l'invention, de l'exemple 1.

### Exemples 2 à 7 :

Les compositions détaillées ci-après illustrent d'autres exemples de crèmes de brillance conformes à l'invention.

### Exemple 2 :

| | Pourcentage massique |
|---|---|
| Methylheptylcyanoacrylate (1) | 10% |
| Cyclopentasiloxane | 44.55% |
| Cyclopentasiloxane dimethicone copolyol | 45% |
| Acide acétique | 0,25 % |
| Parfum | 0,2 % |
| (1) commercialisé par la société Chemence | |

### Exemple 3 :

| | Pourcentage massique |
|---|---|
| Ethylhexyleyanoacrylate (1) | 10% |
| Cyclopentasiloxane | 44.55% |
| Cyclopentasiloxane dimethicone copolyol | 45% |
| Acide acétique | 0.25 % |
| Parfum | 0,2 % |
| (1) O 60 commercialisé par la société Tong Shen | |

### Exemple 4 :

| | Pourcentage massique |
|---|---|
| Methylheptylcyanoacrylate (1) | 9 % |
| Ethylhexylcyanoacrylate (2) | 1 % |
| Cyclopentasiloxane | 44.55% |
| Cyclopentasiloxane dimethicone copolyol | 45% |
| Acide acétique | 0,25 % |
| Parfum | 0,2 % |
| (1) commercialisé par la société Chemence | |
| (2) O-60 commercialisé par la société Tong Shen | |

### Exemple 6 :

| | Pourcentage massique |
|---|---|
| Methylheptylcyanoacrylate (1) | 7 % |
| Butyleyanoacrylate (2) | 3 % |
| Cyclopentasiloxane | 44.55% |
| Cyclopentasiloxane dimethicone copolyol | 45% |
| Acide acétique | 0,25 % |
| Parfum | 0,2 % |
| (1) commercialisé par la société Chemence | |
| (2) B-60 commercialisé par la société Tong Shen | |

## Revendications

1. Composition pour le traitement cosmétique des cheveux comprenant au moins un monomère électrophile répondant la formule (V) : 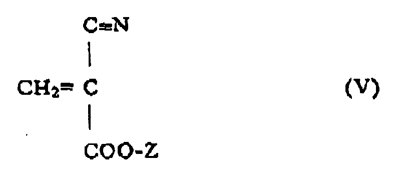 dans laquelle : Z=-(CH₂)₇-CH₃,
-CH(CH₃)-(CH₂)₅CH₃,
-CH₂-CH(C₂H₅)-(CH₂)₃-CH₃,
-(CH₂)₅-CH(CH₃)-CH₃,
-(CH₂)₄-CH(C₂H₅)-CH₃,
et au moins un acide organique non-réducteur choisi parmi l'acide acétique, l'acide formique, l'acide propionique, l'acide butyrique, les acides mono-, di- ou trichloroacétiques, l'acide trifluoroacétique, , dans un milieu cosmétiquement acceptable anhydre choisi parmi, les silicones, les huiles minérales, les huiles végétales, les alcanes en C₅-C₁₀, l'acétone, la méthyléthylcétone, le diméthoxyéthane, le diéthoxyéthane, les alcools gras en C₁₀-C₃₀, les amides gras en C₁₀-C₃₀, les esters d'alcool gras en C₁₀-C₃₀ et leurs mélanges.

2. Composition selon la revendication 1, **caractérisée en ce que** l'acide est l'acide acétique.

3. Composition selon l'une quelconque des revendications précédentes, **caractérisée en ce que** l'acide est contenu en une quantité comprise entre 0,001 et 20%, de préférence entre 0,002 et 10% en poids par rapport au poids total de la composition.

4. Composition selon l'une quelconque des revendications précédentes, **caractérisée en ce que** la quantité de monomère électrophile va de 0,001 à 80 % en poids, de préférence de 0,002 à 75 % en poids, encore plus préférentiellement de 0,1 à 40 % en poids par rapport au poids total de la composition.

5. Composition selon l'une quelconque des revendications précédentes, **caractérisée en ce que** le milieu cosmétiquement acceptable est choisi parmi les huiles d'olive, de ricin, de colza, de coprah, de germes de blé, d'amande douce, d'avocat, de macadania, d'abricot, de carthame, de noix de bancoulier, de camélina, de tamanu, de citron ; l'huile de polybutène, le mélange cyclopentasiloxane/polydiméthylsiloxane dihydroxylé en positions α et ω, ou leurs mélanges.

6. Composition selon l'une quelconque des revendications précédentes, **caractérisée en ce que** la composition contient des inhibiteurs de polymérisation.

7. Composition selon la revendication 6, **caractérisée en ce que** les inhibiteurs sont des inhibiteurs de polymérisation anioniques et/ou radicalaires.

8. Composition selon la revendication 6 ou 7, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfolène et leurs mélanges.

9. Composition selon l'une quelconque des revendications 6 à 8, **caractérisée en ce que** le ou les inhibiteurs de polymérisation sont présents en une quantité allant de 10 ppm à 20 % par rapport au poids total de la composition.

10. Composition selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensioactifs anioniques, cationiques, non ioniques ou amphotères, les polymères fixants ou non, les polyols, les protéines, des vitamines, les colorants directs ou d'oxydation, les agents nacrants, les propulseurs et les agents épaississants minéraux ou organiques.

11. Composition selon la revendication 10, **caractérisée en ce que** l'agent est encapsulé.

12. Utilisation de la composition selon l'une quelconque des revendications précédentes, pour le traitement cosmétique des cheveux.

13. Utilisation selon la revendication 12, en présence d'un agent nucléophile.

14. Utilisation selon la revendication 13, **caractérisée en ce que** l'agent nucléophile est choisi parmi les composés moléculaires, les oligomères, les dendrimères ou polymères possédant des fonctions nucléophiles choisies parmi : R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

15. Utilisation selon la revendication 14, **caractérisée en ce que** l'agent nucléophile est constitué d'ions hydroxyle, notamment ceux présents dans l'eau.

16. Procédé de traitement cosmétique des cheveux, comprenant l'application sur les cheveux d'une composition selon l'une quelconque des revendications 1 à 11 en présence d'un agent nucléophile.

17. Procédé selon la revendication 16, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃-, ClO₄⁻ et H₂O, Ph représentant le groupe phényle, Ar représentant un groupe aryle et R représentant un groupe alkyle en C₁-C₁₀.

18. Procédé selon la revendication 16, **caractérisé en ce que** l'agent nucléophile est constitué d'ions hydroxyle, notamment ceux présents dans l'eau.

19. Procédé selon l'une quelconque des revendications 16 à 18, **caractérisé en ce que** l'on applique la composition sur les cheveux préalablement humidifiés à l'aide d'une solution aqueuse dont le pH a été ajusté à l'aide d'une base, d'un acide ou d'un mélange acide/base.

20. Procédé selon l'une quelconque des revendications 16 à 18, **caractérisé en ce que** les cheveux sont pré-imprégnées à l'aide d'un agent nucléophile différent de l'eau.

21. Procédé selon l'une quelconque des revendications 16 à 18, **caractérisé en ce que** les cheveux sont préalablement réduits avant application de la composition.

22. Procédé selon l'une des revendications 16 à 21, **caractérisé en ce que** l'application de la composition est suivie d'un rinçage.

23. Kit comprenant une première composition contenant au moins un monomère électrophile tel que défini dans la revendication 1, et éventuellement au moins un inhibiteur de polymérisation anionique et/ou radicalaire, ainsi qu'une deuxième composition comprenant, dans un milieu cosmétiquement acceptable anhydre choisi parmi les huiles organiques, les silicones, les huiles minérales, les huiles végétales, les cires, les alcanes en C₅-C₁₀, l'acétone, la méthyléthylcétone, les esters d'acides en C₁-C₂₀ et d'alcools en C₁-C₈, le diméthoxyéthane, le diéthoxyéthane, les alcools gras en C₁₀-C₃₀, les acides gras en C₁₀-C₃₀, les amides gras en C₁₀-C₃₀, les esters d'alcool gras en C₁₀₋C₃₀ et leurs mélanges, au moins un acide organique non-réducteur choisi parmi l'acide acétique, l'acide formique, l'acide propionique, l'acide buturique, les acides mono-, di- ou trichloroacétiques, l'acide trifluoroacétique.

## Claims

1. Composition for the cosmetic treatment of the hair, comprising at least one electrophilic monomer conforming to the formula (V): 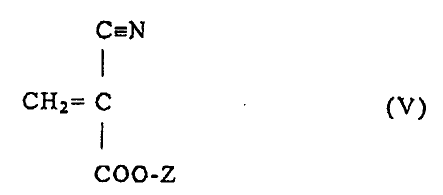 in which: Z = - (CH₂)₇-CH₃,
- CH(CH₃)-(CH₂)₅-CH₃,
- CH₂-CH(C₂H₅)-(CH₂)₃₋CH₃,
- (CH₂)₅-CH(CH₃)-CH₃,
- (CH₂)₄-CH(C₂H₅)-CH₃,
and at least one non-reducing organic acid chosen from acetic acid, formic acid, propionic acid, butyric acid, mono-, di- or trichloroacetic acid and trifluoroacetic acid, in a cosmetically acceptable anhydrous medium chosen from silicones, mineral oils, plant oils, C₅-C₁₀ alkanes, acetone, methyl ethyl ketone, dimethoxyethane, diethoxyethane, C₁₀-C₃₀ fatty alcohols, C₁₀-C₃₀ fatty amides, esters of C₁₀-C₃₀ fatty alcohols, and mixtures thereof.

2. Composition according to Claim 1, **characterized in that** the acid is acetic acid.

3. Composition according to either of the preceding claims, **characterized in that** the acid is present in an amount between 0.001% and 20% by weight, preferably between 0.002% and 10% by weight relative to the total weight of the composition.

4. Composition according to any one of the preceding claims, **characterized in that** the amount of electrophilic monomer ranges from 0.001 to 80% by weight, preferably from 0.002 to 75% by weight, even more preferably from 0.1 to 40% by weight relative to the total weight of the composition.

5. Composition according to any one of the preceding claims, **characterized in that** the cosmetically acceptable medium is chosen from olive oil, castor oil, rapeseed oil, coconut oil, wheatgerm oil, sweet almond oil, avocado oil, macadamia oil, apricot oil, safflower oil, candlenut oil, camelina oil, tamanu oil and lemon oil; polybutene oil, and the cyclopentasiloxane/α, ω-dihydroxylated polydimethylsiloxane mixture, or mixtures thereof.

6. Composition according to any one of the preceding claims, **characterized in that** the composition comprises polymerization inhibitors.

7. Composition according to Claim 6, **characterized in that** the inhibitors are free-radical and/or anionic polymerization inhibitors.

8. Composition according to Claim 6 or 7, **characterized in that** the polymerization inhibitors are selected from sulphur dioxide, nitric oxide, lactone, boron trifluoride, hydroquinone and its derivatives such as hydroquinone monoethyl ether, tert-butylhydroquinone (TBHQ), benzoquinone and its derivatives such as duroquinone, catechol and its derivatives such as tert-butylcatechol and methoxycatechol, anisole and its derivatives such as methoxyanisole, hydroxyanisole or butylated hydroxyanisole, pyrogallol, 2,4-dinitrophenol, 2,4,6-trihydroxybenzene, p-methoxyphenol, hydroxybutyltoluene, alkyl sulphates, alkyl sulphites, alkyl sulphones, alkyl sulphoxides, alkyl sulphides, mercaptans, 3-sulpholene, and mixtures thereof.

9. Composition according to any one of Claims 6 to 8, **characterized in that** the polymerization inhibitor(s) is (are) present in amounts ranging from 10 ppm to 20% relative to the total weight of the composition.

10. Composition according to any one of the preceding claims, **characterized in that** the composition further comprises at least one agent selected from reducing agents, fats, plasticizers, softeners, antifoams, moisturizers, pigments, clays, mineral fillers, UV filters, mineral colloids, peptizers, solubilizers, perfumes, preservatives, anionic, cationic, nonionic or amphoteric surfactants, fixative or non-fixative polymers, polyols, proteins, vitamins, direct dyes or oxidation dyes, pearlizers, propellants and organic or inorganic thickeners.

11. Composition according to Claim 10, **characterized in that** the agent is encapsulated.

12. Use of the composition according to any one of the preceding claims for the cosmetic treatment of the hair.

13. Use according to Claim 12 in the presence of a nucleophile.

14. Use according to Claim 13, **characterized in that** the nucleophile is selected from molecular compounds, oligomers, dendrimers or polymers possessing nucleophilic functions selected from: R₂N⁻, NH₂⁻, Ph₃C⁻, R₃C⁻, PhNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ and H₂O, where Ph represents the phenyl group, Ar represents an aryl group and R represents a C₁-C₁₀ alkyl group.

15. Use according to Claim 14, **characterized in that** the nucleophile is composed of hydroxyl ions, in particular those present in water.

16. Method of cosmetic treatment of the hair, comprising applying a composition according to any one of Claims 1 to 11 to the hair in the presence of a nucleophile.

17. Method according to Claim 16, **characterized in that** the nucleophile is selected from molecular compounds, oligomers, dendrimers or polymers possessing nucleophilic functions selected from: R₂N⁻, NH₂⁻, Ph₃C⁻, R₃C⁻, PhNH⁻, pyridine, ArS⁻, R-C≡C, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ and H₂O, where Ph represents the phenyl group, Ar represents an aryl group and R represents a C₁-C₁₀ alkyl group.

18. Method according to Claim 16, **characterized in that** the nucleophile is composed of hydroxyl ions, in particular those present in water.

19. Method according to any one of Claims 16 to 18, **characterized in that** the composition is applied to the hair which has been wetted beforehand with an aqueous solution whose pH has been adjusted by means of a base, an acid or an acid/base mixture.

20. Method according to any one of Claims 16 to 18, **characterized in that** the hair is preimpregnated with a nucleophile other than water.

21. Method according to any one of Claims 16 to 18, **characterized in that** the hair is prereduced before the composition is applied.

22. Method according to one of Claims 16 to 21, **characterized in that** application of the composition is followed by rinsing.

23. Kit comprising a first composition comprising at least one electrophilic monomer as defined in Claim 1 and optionally at least one free-radical and/or anionic polymerization inhibitor, and a second composition comprising in a cosmetically acceptable anhydrous medium chosen from organic oils, silicones, mineral oils, plant oils, waxes, C₅-C₁₀ alkanes, acetone, methyl ethyl ketone, C₁-C₂₀ acid esters of C₁-C₈ alcohols, dimethoxyethane, diethoxyethane, C₁₀-C₃₀ fatty alcohols, C₁₀-C₃₀ fatty acids, C₁₀-C₃₀ fatty amides, esters of C₁₀-C₃₀ fatty alcohols, and mixtures thereof, at least one non-reducing organic acid chosen from acetic acid, formic acid, propionic acid, butyric acid, mono-, di- or trichloroacetic acid and trifluoroacetic acid.

## Patentansprüche

1. Zusammensetzung für die kosmetische Behandlung der Haare, die mindestens ein elektrophiles Monomer, das der folgenden Formel (V) entspricht: 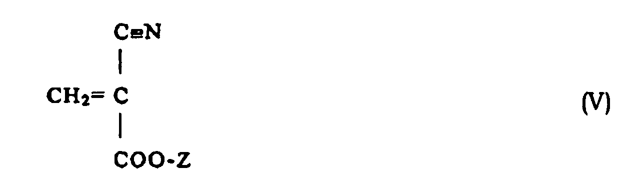 in der: Z = -(CH₂)₇-CH₃,
- CH(CH₃)-(CH₂)₅-CH₃,
- CH₂-CH(C₂H₅)-(CH₂)₃-CH₃,
- (CH₂)₅-CH(CH₃)-CH₃,
- (CH₂)₄-CH(C₂H₅)-CH₃,
und mindestens eine nichtreduzierende organische Säure, die unter Essigsäure, Ameisensäure, Propionsäure, Buttersäure, Mono-, Di- oder Trichloressigsäure und Trifluoressigsäure ausgewählt ist, in einem kosmetisch akzeptablen wasserfreien Medium enthält, das unter den Siliconen, Mineralölen, pflanzlichen Ölen, C₅₋₁₀-Alkanen, Aceton, Methylethylketon, Dimethoxyethan, Diethoxyethan, C₁₀₋₃₀-Fettalkoholen, C₁₀₋₃₀-Fettamiden, C₁₀₋₃₀-Fettalkoholestern und deren Gemischen ausgewählt ist.

2. Zusammensetzung nach Anspruch 1, **dadurch gekennzeichnet, dass** die Säure Essigsäure ist.

3. Zusammensetzung nach einem der vorhergehenden Ansprüche,
**dadurch gekennzeichnet, dass** die Säure in einem Mengenanteil von 0,001 bis 20 Gew.-% und vorzugsweise 0,002 bis 10 Gew.-%, bezogen auf das Gesamtgewicht der Zusammensetzung, enthalten ist.

4. Zusammensetzung nach einem der vorhergehenden Ansprüche,
**dadurch gekennzeichnet, dass** der Mengenanteil des elektrophilen Monomers im Bereich von 0,001 bis 80 Gew.-%, vorzugsweise von 0,002 bis 75 Gew.-% und noch bevorzugter von 0,1 bis 40 Gew.-%, bezogen auf das Gesamtgewicht der Zusammensetzung, liegt.

5. Zusammensetzung nach einem der vorhergehenden Ansprüche,
**dadurch gekennzeichnet, dass** das kosmetisch akzeptable Medium unter Olivenöl, Ricinusöl, Rapsöl, Kopraöl, Weizenkeimöl, Süßmandelöl, Avocadoöl, Macadamiaöl, Aprikosenkernöl, Distelöl, Kukuinussöl, Leinöl, Tamanuöl, Zitronenöl; Polybutenöl, dem Gemisch aus Cyclopentasiloxan/in α-und ω-Stellung dihydroxylierten Polydimethylsiloxan, oder deren Gemischen ausgewählt ist.

6. Zusammensetzung nach einem der vorhergehenden Ansprüche,
**dadurch gekennzeichnet, dass** die Zusammensetzung Polymerisationsinhibitoren enthält.

7. Zusammensetzung nach Anspruch 6, **dadurch gekennzeichnet,**
**dass** die Inhibitoren anionische und/oder radikalische Polymerisationsinhibitoren sind.

8. Zusammensetzung nach Anspruch 6 oder 7, die Polymerisationsinhibitoren unter Schwefeldioxid, Stickoxid, Lacton, Bortrifluorid, Hydrochinon und seinen Derivaten, wie Hydrochinonmonoethylether, t-Butylhydrochinon (TBHQ), Benzochinon und seinen Derivaten, wie Durochinon, Catechin und seinen Derivaten, wie t-Butylcatechin und Methoxycatechin, Anisol und seinen Derivaten, wie Methoxyanisol, Hydroxyanisol oder Butylhydroxyanisol, Pyrogallol, 2,4-Dinitrophenol, 2,4,6-Trihydroxybenzol, p-Methoxyphenol, Hydroxybutyltoluol, Alkylsulfaten, Alkylsulfiten, Alkylsulfones, Alkylsulfoxiden, Alkylsulfiden, Mercaptanen, 3-Sulfolen und deren Gemischen ausgewählt ist.

9. Zusammensetzung nach einem der Ansprüche 6 bis 8, **dadurch gekennzeichnet, dass** der oder die Polymerisationsinhibitoren in einer Menge von 10 ppm bis 20 %, bezogen auf das Gesamtgewicht der Zusammensetzung, enthalten sind.

10. Zusammensetzung nach einem der vorhergehenden Ansprüche,
**dadurch gekennzeichnet, dass** die Zusammensetzung ferner mindestens einen Stoff enthält, der unter den Reduktionsmitteln, Fettsubstanzen, Weichmachern, beruhigenden Stoffen, Schaumverhütungsmitteln, Hydratisierungsmitteln, Pigmenten, Tonen, anorganischen Füllstoffen, UV-Filtern, anorganischen Kolloiden, peptisierenden Stoffen, Lösungsvermittlern, Parfums, Konservierungsmitteln, anionischen, kationischen, nichtionischen oder amphoteren grenzflächenaktiven Stoffen, festigenden oder nicht festigenden Polymeren, Polyolen, Proteinen, Vitaminen, Direktfarbstoffen oder Oxidationsfarbstoffen, Perlglanzmitteln, Treibmitteln und anorganischen oder organischen Verdickungsmitteln ausgewählt ist.

11. Zusammensetzung nach Anspruch 10, **dadurch gekennzeichnet, dass** der Stoff eingekapselt ist.

12. Verwendung der Zusammensetzung nach einem der vorhergehenden Ansprüche für die kosmetische Behandlung der Haare.

13. Verwendung nach Anspruch 12 in Gegenwart eines nucleophilen Stoffes.

14. Verwendung nach Anspruch 13, **dadurch gekennzeichnet, dass** der nucleophile Stoff unter den Molekülen, Oligomeren, Dendrimeren oder Polymeren ausgewählt ist, die nucleophile Funktionen aufweisen, die unter R₂N⁻, NH₂^{-,} Ph₃C^{-*,*} R₃C^{-,} PhNH^{-,} Pyridin, ArS^{-,} R-C≡C^{-,} RS^{-,} SH^{-,} RO^{-,} R₂NH, ArO^{-,} N₃^{-,} OH^{-,} ArNH₂, NH₃, I^{-,} Br^{-,} Cl^{-,} RCOO^{-,} SCN^{-,} ROH, RSH, NCO^{-*,*} CN^{-,} NO₃^{-,} ClO₄⁻ und H₂O ausgewählt sind, wobei Ph die Phenylgruppe bedeutet ; Ar eine Arylgruppe bedeutet und R eine C₁₋₁₀-Alkylgruppe ist.

15. Verwendung nach Anspruch 14, **dadurch gekennzeichnet, dass** der nucleophile Stoff aus Hydroxid-Ionen besteht, insbesondere solchen, die in Wasser vorliegen.

16. Verfahren zur kosmetischen Behandlung der Haare, das das Aufbringen einer Zusammensetzung nach einem der Ansprüche 1 bis 11 in der Gegenwart eines nucleophilen Stoffes umfasst.

17. Verfahren nach Anspruch 16, **dadurch gekennzeichnet, dass** der nucleophile Stoff unter den Molekülen, Oligomeren, Dendrimeren oder Polymeren ausgewählt ist, die nucleophile Funktionen aufweisen, die unter R₂N^{-,} NH₂^{-,} Ph₃C^{-,} R₃C^{-,} PhNH^{-,} Pyridin, ArS^{-,} R-C≡C^{-,} RS^{-,} SH^{-,} RO^{-,} R₂NH, ArO^{-,} N₃^{-,} OH^{-,} ArNH₂, NH₃, I^{-,} Br^{-,} Cl^{-,} RCOO^{-,} SCN^{-,} ROH, RSH, NCO^{-,} CN^{-,} NO₃^{-,} ClO₄⁻ und H₂O ausgewählt sind, wobei Ph die Phenylgruppe bedeutet ; Ar eine Arylgruppe bedeutet und R eine C₁₋₁₀-Alkylgruppe ist.

18. Verfahren nach Anspruch 16, **dadurch gekennzeichnet, dass** der nucleophile Stoff aus Hydroxid-Ionen besteht, insbesondere solchen, die in Wasser vorliegen.

19. Verfahren nach einem der Ansprüche 16 bis 18, **dadurch gekennzeichnet, dass** die Zusammensetzung auf die Haare aufgebracht wird, die vorab mit Hilfe einer wässrigen Lösung befeuchtet wurden, deren pH-Wert mit Hilfe einer Base, einer Säure oder einem Säure/Base-Gemisch eingestellt worden ist.

20. Verfahren nach einem der Ansprüche 16 bis 18, **dadurch gekennzeichnet, dass** die Haare vorab mit Hilfe eines nucleophilen Stoffes, der von Wasser verschieden ist, getränkt wurden.

21. Verfahren nach einem der Ansprüche 16 bis 18, **dadurch gekennzeichnet, dass** die Haare vor dem Aufbringen der Zusammensetzung vorab reduziert wurden.

22. Verfahren nach einem der Ansprüche 16 bis 21, **dadurch gekennzeichnet, dass** nach dem Aufbringen der Zusammensetzung ein Spülschritt folgt.

23. Kit, der eine erste Zusammensetzung, die mindestens ein elektrophiles Monomer, wie es in Anspruch 1 definiert ist, und gegebenenfalls mindestens einen anionischen und/oder radikalischen Polymerisationsinhibitor enthält, sowie eine zweite Zusammensetzung umfasst, die in einem kosmetisch akzeptablen wasserfreien Medium, das unter den organischen Ölen, Siliconen, Mineralölen, pflanzlichen Ölen, Wachsen, C₅₋₁₀-Alkanen, Aceton, Methylethylketon, Estern von C₁-₂₀-Säuren und C₁₋₈-Alkoholen, Dimethoxyethan, Diethoxyethan, C₁₀₋₃₀-Fettalkoholen, C₁₀₋₃₀-Fettsäuren, C₁₀₋₃₀-Fettamiden, C₁₀₋₃₀-Fettalkoholestern und deren Gemischen ausgewählt ist, mindestens eine nichtreduzierende organische Säure enthält, die unter Essigsäure, Ameisensäure, Propionsäure, Buttersäure, Mono-, Di- oder Trichloressigsäure und Trifluoressigsäure ausgewählt ist.
